# EUROPÄISCHE PATENTSCHRIFT

(11) **EP 2 151 279 B1**
(45) Veröffentlichungstag und Bekanntmachung des Hinweises auf die Patenterteilung: **13.02.2013**
(21) Anmeldenummer: 09009984.7
(22) Anmeldetag: 03.08.2009
(51) Int. Cl.: B03B 9/06, B03B 13/00, C02F 3/00, C02F 3/28, C10L 3/08, C12P 5/02

(54) **Verfahren zum Betrieb einer mechanisch-biologischen Abfallbehandlungsanlage mit einem Prozesswasserkreislauf**
Method for operating a mechanical-biological waste treatment assembly with a process water circuit
Procédé de fonctionnement d'une installation de traitement des déchets mécanique-biologique dotée d'un circuit d'eau de traitement

(30) Priorität: 04.08.2008 DE 102008036087
(43) Veröffentlichungstag der Anmeldung: 10.02.2010
(73) Patentinhaber: Zweckverband Abfallbehandlung Kahlenberg, 77975 Ringsheim (DE)
(72) Erfinder: Gibis, Georg, 77975 Ringsheim (DE); Sauter, Tobias, 77977 Rust (DE)
(74) Vertreter: Otten, Herbert

(56) Entgegenhaltungen:
- DE-A1- 19 803 140
- JP-A- 2003 039 039
- JP-A- 2005 152 875

## Beschreibung

Die Erfindung betrifft ein Verfahren gemäß dem Oberbegriff des Anspruchs 1,

Aus der DE 198 03 140 A1 ist ein Verfahren zum Betrieb einer mechanisch-biologischen Abfallbehandlungsanlage bzw. zur stofflichen Verwertung von Restabfällen bekannt. Weiterhin ist aus der JP 2003 039039 A ein Behandlungssystem bekannt, bei dem bei der Fermentation die Ammonium-Konzentration im Prozesswasser durch Zugabe von Verdünnungswasser so eingeregelt wird, dass eine Inhibierung der Biogasproduktion durch zu hohe Ammonium-Konzentration verhindert wird. Schließlich ist aus der JP 2005 152875 A ein Behandlungsverfahren bekannt, bei dem bei der Fermentation die elektrische Leitfähigkeit im Prozesswasser durch Zugabe von Verdünnungswasser so eingeregelt wird, dass ein vorbestimmter Wert nicht überschritten wird.

Es ist Aufgabe der Erfindung, ein Verfahren zum Betrieb einer mechanisch-biologischen Abfallbehandlungsanlage mit einem Prozesswasserkreislauf vorzuschlagen, bei welchem durch optimierte Verfahrensabläufe ein reibungsloser Betrieb mit einem steten und hohen Gasertrag und/oder einer hohen Festbrennstoffqualität gesichert ist.

Diese Aufgabe wird ausgehend von den Merkmalen des Oberbegriffs des Anspruchs 1 durch die kennzeichnenden Merkmale des Anspruchs 1 gelöst. In den Unteransprüchen sind vorteilhafte und zweckmäßige Weiterbildungen angegeben.

Bei dem erfindungsgemäßen Verfahren zum Betrieb einer mechanisch-biologischen Abfallbehandlungsanlage (MBA) mit einem Prozesswasserkreislauf, in welchem ein Prozesswasserüberschuss produziert wird, wird abhängig von einem ermittelten Salzgehalt des Prozesswassers dem Prozesswasserkreislauf Flüssigkeit zugeführt und wird zur Erniedrigung der Chloridkonzentration dem Prozesswasserkreislauf, insbesondere in der Fermentationseinrichtung, bei paralleler Reduktion der Zugabe von Eisen(III)-chlorid eine Zugabe von Eisenoxid als Entschwefelungsmittel erhöht, um einen Eintrag von Chloriden aus dem Prozesswasser in den Feststoff zu vermindern, und wird abhängig von einem ermittelten Stickstoffgehalt des Prozesswassers dem Prozesswasserkreislauf Flüssigkeit zugeführt, um den Stickstoffgehalt des Prozesswassers für den Betrieb der Fermentationseinrichtung zu senken. Ein derartiges Überwachen des Prozesswasserkreislaufes und ein hierauf basierendes kontrollierendes bzw. regelndes Eingreifen in den Prozesswasserkreislauf sichert eine stetige, hohe und gleichbleibende Gasproduktion in der Fermentereinrichtung, da die Mikroorganismen vor Überlastungen geschützt werden, bzw. sichert eine hohe und gleichbleibende Qualität des erzeugten Festbrennstoffes, da eine Erhöhung des beim Festbrennstoff unerwünschten Chloridgehalts vermieden wird.

Bei dem erfindungsgemäßen Verfahren zum Betrieb einer mechanisch-biologischen Abfallbehandlungsanlage ist es weiterhin vorgesehen, im Prozesswasserkreislauf als Stickstoffgehalt den Ammoniumstickstoffgehalt des Prozesswassers zu bestimmen, wobei dem Prozesswasserkreislauf insbesondere vor oder in der Fermentationseinrichtung ggf. Flüssigkeit zugeführt wird. Da Ammoniumstickstoff in höheren Konzentrationen hemmend auf die Biogasbildung wirkt, gewährleistet eine derartige Überwachung des Ammoniumstickstoffgehalts im Fermenter eine hohe Biogasproduktion. Insbesondere kann durch eine Zugabe von Flüssigkeit in die Fermentationseinrichtung der Ammoniumstickstoffgehalt schneller und kostengünstiger korrigiert werden als durch eine biologische Ammoniumstickstoff-Reduktion im Kreislaufwasser.

Bei dem erfindungsgemäßen Verfahren zum Betrieb einer mechanisch-biologischen Abfallbehandlungsanlage ist es auch vorgesehen, den Salzgehalt des Prozesswassers nach der Fermentationseinrichtung und vor der mechanischen Trenneinrichtung zu bestimmen. Da der Salzgehalt im Prozesswasser den Salzgehalt im Festbrennstoff beeinflusst, welcher in der mech. Trenneinrichtung (I) erzeugt wird, ist es wirtschaftlich sinnvoll durch vorgeschaltete Maßnahmen einen hohen Salzgehalt des Festbrennstoffs zu vermeiden. Ein zu hoher Salzgehalt im Brennstoff führt zu erhöhter Kesselkorrosion, z. B. durch Chloride, und zu erhöhten Emissionen beim Verbrennen, z.B. in Form von NOx und SO2, und verursacht somit hohe Folgekosten durch einen erhöhten Verschleiß der Anlagen, welche mit dem Festbrennstoff betrieben werden, oder durch erhöhte Umweltbelastung.

Bei dem erfindungsgemäßen Verfahren zum Betrieb einer mechanisch-biologischen Abfallbehandlungsanlage ist es weiterhin vorgesehen, das Prozesswasser in dem Prozesswasserkreislauf in wenigstens einer Misch- und Speichereinrichtung aufzustauen, um Konzentrationsspitzen insbesondere beim Salzgehalt und/oder beim Stickstoffgehalt zu vermeiden und/oder hydraulische Belastungsspitzen zu vermeiden und/oder einen Gehalt an organischen Stoffen zu reduzieren. Durch die Misch- und Speichereinrichtung ergibt sich als Vorteile, dass eine gleichmäßigere Beschickung der Fermenter mit organischer Fracht zu einer gleichmäßigeren Biogasbildung führt - Biogasmenge pro Zeit bleibt konstant -, wobei hierdurch der in Vergärungsanlagen üblicherweise eingesetzte Gasspeicher zur vergleichmäßigten Beschickung nachgeschalteter Gasmotoren mit Biogas entfallen kann. Durch die Misch- und Speichereinrichtung ergibt sich weiterhin als Vorteile, dass auftretende Konzentrationsspitzen bei Stickstoffgehalt und Salzgehalt, die zu hemmenden Prozessen bei den Mikroorganismen im Fermenter führen können und Abbauprozesse und Biogasbildung negativ beeinflussen, wirksam vermieden werden. Die Vergleichmäßigung mittels der Misch-und Speichereinrichtung verhindert das Auftreten dieser Spitzen und verhindert dadurch auch die beschriebenen negativen Folgeerscheinungen.

Bei dem erfindungsgemäßen Verfahren zum Betrieb einer mechanisch-biologischen Abfallbehandlungsanlage wird die zugeführte Flüssigkeit mit dem Prozesswasser vor einer Einleitung in die Fermentationseinrichtung in einer Misch-und Speichereinrichtung vermischt. Hierdurch werden zu starke Konzentrationsunterschiede im Fermenter durch Vermischung von Flüssigkeit mit Prozesswasser in der vorgeschalteten Misch-und Speichereinrichtung wirksam vermieden.

Bei dem erfindungsgemäßen Verfahren zum Betrieb einer mechanisch-biologischen Abfallbehandlungsanlage ist es auch vorgesehen, den Salzgehalt und/oder den Stickstoffgehalt des Prozesswassers in der Misch- und Speichereinrichtung zu messen. Hierdurch ist der Stickstoffgehalt und/oder der Salzgehalt bekannt, bevor Prozesswasser in den Fermenter gelangt, somit kann der Fermenter optimal betrieben werden, da nicht erst auf eine Zustandsänderung im Fermenter reagiert wird, sondern schon bei der Beschickung des Fermenters auf eine gleichmäßige Biogasproduktion geachtet wird. Erfindungsgemäß ist es auch vorgesehen, die organische Konzentration zu erfassen, um durch Kenntnis dieser Größe in der Misch- und Speichereinrichtung die Zufuhr zum Fermenter zu regeln und die Biogasbildung zu vergleichmäßigen.

Bei dem erfindungsgemäßen Verfahren zum Betrieb einer mechanisch-biologischen Abfallbehandlungsanlage werden dem Prozesswasser Ablagerungsinhibitoren zur Vermeidung von Ablagerungen insbesondere im Prozesswasserkreislauf und insbesondere in den das überschüssige Prozesswasser führenden Rohrleitungen und Aggregaten zugegeben. Hierdurch können Betriebsbeeinträchtigungen durch Ablagerungen in Rohren, insbesondere durch einen reduzierten Durchflussquerschnitt oder einen Verschluss vermieden werden. Auch bei den sonstigen Aggregaten mit mechanisch bewegbaren Teilen wird ein Verschleiß gemindert und das Risiko für einen Funktionsausfall reduziert.

Bei dem erfindungsgemäßen Verfahren zum Betrieb einer mechanisch-biologischen Abfallbehandlungsanlage ist ein Feststoffgehalt des Prozesswassers für einen optimalen Betrieb der Fermentationseinrichtung durch Rückführung von feststoffarmem Prozesswasser aus der Dekantereinheit oder durch Zuführung von eingedickten Reststoffen regelbar. Hierdurch ist der Feststoffgehalt im Fermenter optimal einstellbar. Zwar müssen inertes Sediment und abgestorbene Biomasse aus der Fermentationseinrichtung entfernt werden, hierbei darf aber nicht zuviel aktive Biomasse abgezogen werden. Auch darf der Feststoffgehalt im Fermenter nicht zu groß werden, da sonst der Fermenterinhalt zu dickflüssig und damit schwerer durchmischbar und schwerer pumpfähig wird. Das erforderliche ausgewogene Verhältnis zur Gewährleistung eines bestmöglichen Abbaus kann durch eine dem Regelungsbedarf entsprechende Rückführung bzw. eine dem Regelungsbedarf entsprechende Zuführung aufrechterhalten werden.

Unter einer Flüssigkeit, welche dem Prozesswasserkreislauf zugegeben wird, ist im Sinne der Erfindung Wasser und insbesondere Oberflächenwasser oder schwach belastetes Brauchwasser oder Sickerwasser zu verstehen, welches insbesondere in einer Abfallbehandlungsanlage und/oder einer zugeordneten Deponie gesammelt wird bzw. dort anfällt.

Weitere Einzelheiten der Erfindung werden in der Zeichnung anhand eines schematisch dargestellten Ausführungsbeispiels beschrieben.

Hierbei zeigt:
Figur 1: eine schematische Darstellung einer Abfallbehandlungsanlage mit einem Prozesswasserkreislauf.
Figur 2: eine alternative Ausführungsform der Erfindung.

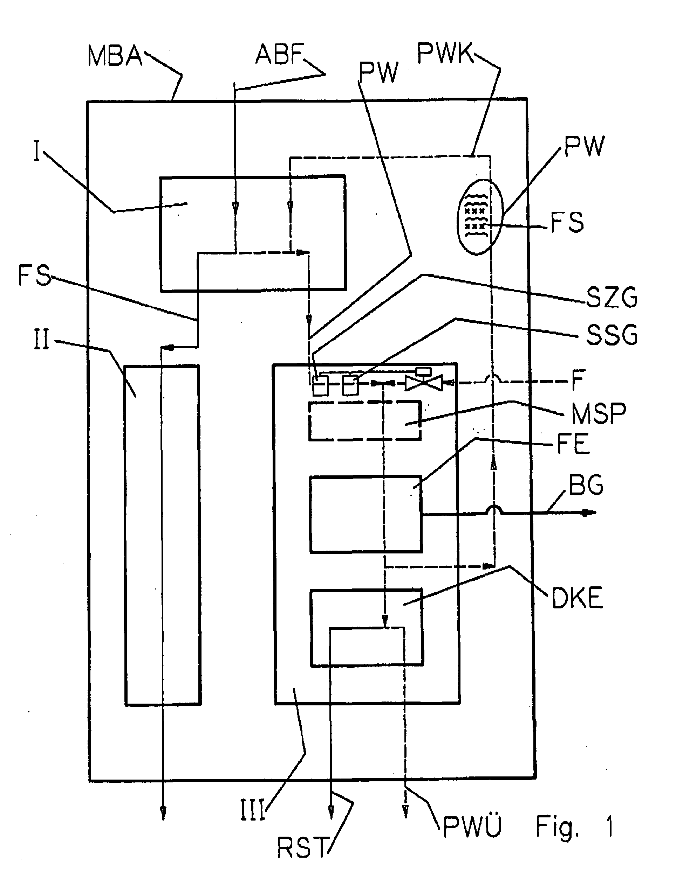

In der Figur 1 ist eine mechanisch-biologische Abfallbehandlungsanlage MBA schematisch dargestellt. Die MBA weist einen Prozesswasserkreislauf PWK auf, wobei in diesem ein Prozesswasserüberschuss PWÜ produziert wird. Dies ist im Wesentlichen dadurch begründet, dass über den Flüssigkeitsanteil im behandelten Abfall ABF dem Prozesswasserkreislauf PWK stetig Wasser zugeführt wird. Die MBA umfasst eine mechanische Trenneinrichtung I, in welcher der Abfall ABF in einen überwiegend festen Abfallbestandteil, den Feststoff FS, und einen überwiegend flüssigen, organikhaltigen Abfallbestandteil, das Prozesswasser PW, aufgetrennt wird. Weiterhin umfasst die MBA eine mehrstufige Bearbeitungseinrichtung II, in welcher der Feststoff FS insbesondere zu Festbrennstoff weiterverarbeitet wird. Parallel zu der Bearbeitungseinrichtung II umfasst die MBA eine mehrstufige Bearbeitungseinrichtung III, in welcher das Prozesswasser PW behandelt wird. In der Bearbeitungseinrichtung III wird zunächst aus dem aus der Trenneinrichtung I zuströmenden Prozesswasser PW in einer Fermentationseinrichtung FE Gas bzw. Biogas BG erzeugt. Nach dem Durchlaufen der Fermentationseinrichtung FE wird das Prozesswasser PW wenigstens teilweise in einer Dekantereinrichtung DKE in Reststoffe RST und feststoffarmes Prozesswasser PW getrennt, wobei die Reststoffe RST aus der MBA ausgeschleust oder wenigstens teilweise in der MBA weiterverarbeitet werden und wobei ein überschüssiges Volumen des feststoffarmen Prozesswassers PW als Prozesswasserüberschuss PWÜ aus dem Prozesswasserkreislauf PWK und insbesondere aus der MBA ausgeschleust wird. In einer Variante verbleibt ein restliches Volumen des Prozesswassers PW in dem Prozesswasserkreislauf PWK, insbesondere zur Absenkung des Feststoffgehalts in der Fermentationsstufe (siehe Figur 1). Abhängig von einem regelmäßig gemessenen Salzgehalt SZG des Prozesswassers PW wird dem Prozesswasserkreislauf PWK Flüssigkeit F beispielsweise in Form von gesammeltem Oberflächenwasser zugeführt und wird zur Erniedrigung der Chloridkonzentration dem Prozesswasserkreislauf PWK, insbesondere in der Fermentationseinrichtung FE, bei paralleler Reduktion der Zugabe von Eisen(III)-chlorid eine Zugabe von Eisenoxid als Entschwefelungsmittel erhöht, um einen Eintrag von Salz aus dem Prozesswasser PW in den Feststoff FP zu vermindern. Ergänzend wird abhängig von einem regelmäßig gemessenen Stickstoffgehalt SSG des Prozesswassers PW dem Prozesswasserkreislauf PWK Flüssigkeit F zugeführt, um den Stickstoffgehalt des Prozesswassers PW für den Betrieb der Fermentationseinrichtung FE zu senken. Hierbei wird der Salzgehalt SZG und der Stickstoffgehalt SSG an einer oder mehreren Stellen des Prozesswasserkreislaufs PWK in regelmäßigen Abständen oder fortlaufend gemessen. Insbesondere werden die Messungen derart ausgewertet, dass ein Trend errechnet wird und frühzeitig steuernd bzw. regelnd eingegriffen wird, um den Salzgehalt SZG und den Stickstoffgehalt SSG des Prozesswassers PW in einem vorgegebenen Toleranzbereich zu halten.

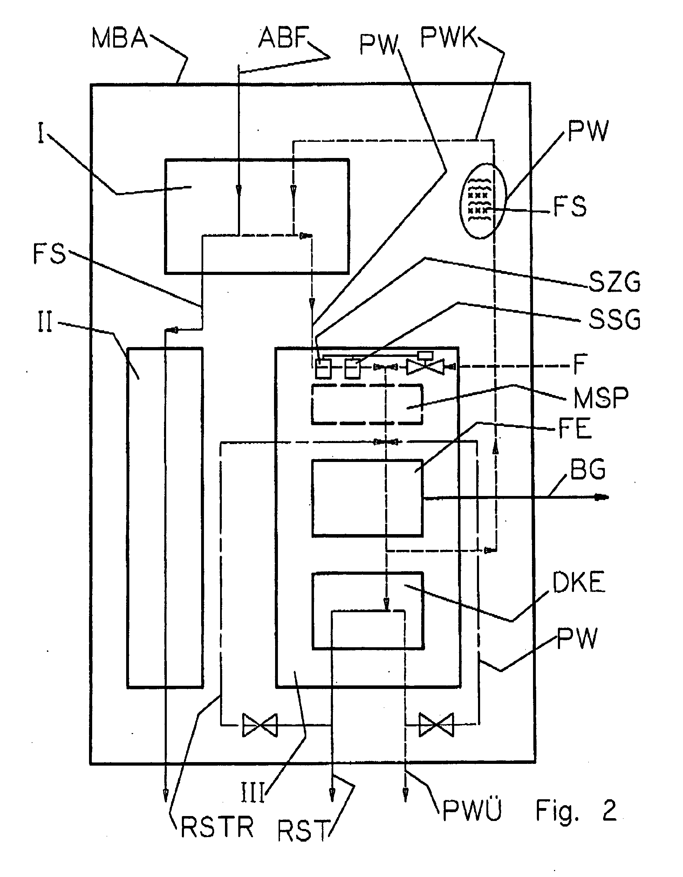

Figur 2 zeigt das aus der Figur 1 bekannte Verfahrensschema zum Betrieb einer mechanisch-biologischen Abfallbehandlungsanlage (MBA), wobei ein Feststoffgehalt des Prozesswassers (PW) für einen optimalen Betrieb der Fermentationseinrichtung (FE) durch Rückführung von feststoffarmem Prozesswasser (PW) aus der Dekantereinheit (DKE) reduzierbar ist. Weiterhin zeigt die Figur 2 die Möglichkeit einer Rückführung von Reststoff RST. Ein Teil des als eingedickter Dekanterschlamm vorliegenden Reststoffs RST kann über eine Reststoffrückleitung RSTR der Fermentationseinrichtung FE zugeführt werden, um bei Bedarf den Feststoffgehalt in der Fermentationseinrichtung FE zu erhöhen.

Die Erfindung ist nicht auf dargestellte oder beschriebene Ausführungsbeispiele beschränkt. Sie umfasst vielmehr Weiterbildungen der Erfindung im Rahmen der Schutzrechtsansprüche.

### Bezugszeichenliste:

- DKE: Dekantereinrichtung
- BG: Gas bzw. Biogas
- F: Flüssigkeit
- FE: Fermentationseinrichtung
- FS: Feststoff bzw. überwiegend fester Abfallbestandteil
- MBA: mechanisch-biologische Abfallbehandlungsanlage
- MSP: Misch- und Speichereinrichtung
- PW: Prozesswasser bzw. überwiegend flüssiger, organikhaltiger Abfallbestandteil
- PWK: Prozesswasserkreislauf
- PWÜ: Prozesswasserüberschuss
- RST: Reststoff
- RSTS: Reststoffrückleitung
- SZG: Salzgehalt
- SSG: Stickstoffgehalt
- I: mechanische Trenneinrichtung
- II: mehrstufige Bearbeitungseinrichtung
- III: mehrstufige Bearbeitungseinrichtung

## Patentansprüche

1. Verfahren zum Betrieb einer mechanisch-biologischen Abfallbehandlungsanlage (MBA) mit einem Prozesswasserkreislauf (PWK), wobei die Abfallbehandlungsanlage (MBA) einen Prozesswasserüberschuss (PWÜ) produziert, umfassend
- eine mechanische Trenneinrichtung (I), in welcher Abfall in einen überwiegend festen Abfallbestandteil, den Feststoff (FS), und einen überwiegend flüssigen, organikhaltigen Abfallbestandteil, das Prozesswasser (PW), aufgetrennt wird,
- eine mehrstufige Bearbeitungseinrichtung (II), in welcher der Feststoff (FS) weiterverarbeitet wird,
- eine mehrstufige Bearbeitungseinrichtung (III), welche parallel zu der βeaxbeitungseinrichtung (II) betrieben wird und in welcher das Prozesswasser (PW) weiterverarbeitet wird,
- wobei in der Bearbeitungseinrichtung (III) zunächst aus dem aus der Trenneinrichtung (I) zuströmenden Prozesswasser (PW) in einer Fermentationseinrichtung (FE) Gas (BG) erzeugt wird,
- wobei das Prozesswasser (PW) nach dem Durchlaufen der Fermentationseinrichtung (FE) wenigstens teilweise in einer Dekantereinrichtung (DKE) in Reststoffe (RST) und feststoffarmes Prozesswasser (PW) getrennt wird,
- wobei die Reststoffe aus der MBA ausgeschleust oder wenigstens teilweise in der MBA weiterverarbeitet werden,
- wobei ein überschüssiges Volumen (PWÜ) des feststoffarmen Prozesswassers (PW) aus dem Prozesswasserkreislauf (PWK) und insbesondere aus der MBA ausgeschleust wird,
- wobei ein restliches Volumen des feststoffarmen Prozesswasser (PW) in dem Prozesswasserkreislauf (PWK) verbleibt,
**dadurch gekennzeichnet,**
**dass** abhängig von einem ermittelten Salzgehalt (SZG) des Prozesswassers (PW) dem Prozesswasserkreislauf (PWK) Flüssigkeit (F) zugeführt wird und zur Erniedrigung der Chloridkonzentration dem Prozesswasserkreislauf (PWK) bei paralleler Reduktion der Zugabe von Eisen(III)-chlorid eine Zugabe von Eisenoxid als Entschwefelungsmittel erhöht wird, um einen Eintrag von Salz aus dem Prozesswasser (PW) in den Feststoff (FP) zu vermindern,
und abhängig von einem ermittelten Stickstoffgehalt (SSG) des Prozesswassers (PW) dem Prozesswasserkreislauf (PWK) Flüssigkeit (F) zugeführt wird, um den Stickstoffgehalt des Prozesswassers (PW) für den Betrieb der Fermentationseinrichtung (FE) zu senken.

2. Verfahren zum Betrieb einer mechanisch-biologischen Abfallbehandlungsanlage (MBA) nach Anspruch 1, **dadurch gekennzeichnet, dass** im Prozesswasserkreislauf (PWK) als Stickstoffgehalt (SSG) der Ammoniumstickstoffgehalt des Prozesswassers (PW) bestimmt wird, wobei dem Prozesswasserkreislauf (PWK) insbesondere vor oder in der Fermentationseinrichtung (FE) ggf. Flüssigkeit (F) zugeführt wird.

3. Verfahren zum Betrieb einer mechanisch-biologischen Abfallbehandlungsanlage (MBA) nach Anspruch 1, **dadurch gekennzeichnet, dass** der Salzgehalt (SZG) des Prozesswassers (PW) nach der Fermentationseinrichtung (FE) und vor der mechanischen Trenneinrichtung (I) bestimmt wird.

4. Verfahren zum Betrieb einer mechanisch-biologischen Abfallbehandlungsanlage (MBA) nach einem der vorhergehenden Ansprüche, **dadurch gekennzeichnet, dass** das Prozesswassers (PW) in dem Prozesswasserkreislauf (PWK) in wenigstens einer Misch- und Speichereinrichtung (MSP) aufgestaut wird, um Konzentrationsspitzen insbesondere beim Salzgehalt (SZG) und/oder beim Stickstoffgehalt (SSG) und/oder hydraulische Belastungsspitzen und/oder einen Gehalt an organischen Stoffen zu reduzieren.

5. Verfahren zum Betrieb einer mechanisch-biologischen Abfallbehandlungsanlage (MBA) nach einem der vorhergehenden Ansprüche, **dadurch gekennzeichnet, dass** die zugeführte Flüssigkeit (F) mit dem Prozesswasser (PW) vor einer Einleitung in die Fermentationseinrichtung (FE) in einer Misch- und Speichereinrichtung (MSP) vermischt wird.

6. Verfahren zum Betrieb einer mechanisch-biologischen Abfallbehandlungsanlage (MBA) nach einem der vorhergehenden Ansprüche, **dadurch gekennzeichnet, dass** der Salzgehalt (SZG) und/oder der Stickstoffgehalt (SSG) des Prozesswassers (PW) in der Misch- und Speichereinrichtung (MSP) gemessen wird.

7. Verfahren zum Betrieb einer mechanisch-biologischen Abfallbehandlungsanlage (MBA) nach einem der vorhergehenden Ansprüche, **dadurch gekennzeichnet, dass** dem Prozesswasser (PW) Ablagerungsinhibitoren zur Vermeidung von Ablagerungen insbesondere im Prozesswasserkreislauf (PWK) und insbesondere in den das überschüssige Prozesswassers (PW) führenden Rohrleitungen und Aggregaten zugegeben werden.

8. Verfahren zum Betrieb einer mechanisch-biologischen Abfallbehandlungsanlage (MBA) nach einem der vorhergehenden Ansprüche, **dadurch gekennzeichnet, dass** ein Feststoffgehalt des Prozesswassers (PW) für einen optimalen Betrieb der Fermentationseinrichtung (FE) durch Rückführung von feststoffarmem Prozesswasser (PW) aus der Dekantereinheit (DKE) oder durch Zuführung von eingedickte Reststoffen regelbar ist.

## Claims

1. Method for operating a mechanical-biological waste treatment system (MBA) with a processing water circuit (PWK), wherein the waste treatment system (MBA) produces an excess of processing water (PWÜ), comprising
- a mechanical separating device (I), in which waste is separated into a predominantly solid waste component, solid matter (FS), and a predominantly liquid, organic-holding waste component, processing water (PW),
- a multiple stage processing device (II) in which the solid matter (FS) is processed further,
- a multiple stage processing device (III) which is operated in parallel to the processing device (II) and in which the processing water (PW) is further processed,
- wherein gas (BG) is produced in the processing device (III) firstly from the processing water (PW) flowing from the separating device (I) in a fermentation device (FE),
- wherein the processing water (PW) after running through the fermentation device (FE) is separated at least partly in a decanter device (DKE) into residual matter (RST) and processing water (PW) containing low amounts of solid matter,
- wherein the residual matter is discharged out of the MBA or is processed further at least partly in the MBA,
- wherein an excess volume (PWÜ) of the processing water (PW) with a low solids content is discharged out of the processing water circuit (PWK) and in particular out of the MBA,
- wherein a residual volume of the processing water (PW) with a low solids content remains in the processing water circuit (PWK),
**characterised in that**
depending on the determined salt content (SZG) of the processing water (PW) liquid (F) is supplied to the processing water circuit (PWK), and to reduce the chloride concentration the addition of iron oxide as a desulfurisation agent is increased to the processing water circuit (PWK) with a parallel reduction of the addition of iron(III) chloride in order to reduce the intake of salt from the processing water (PW) into the solid matter (FP), and depending on the determined nitrogen content (SSG) of the processing water (PW) liquid (F) is supplied to the processing water circuit (PWK) in order to reduce the content of nitrogen in the processing water (PW) for the operation of the fermentation device (FE).

2. Method for operating a mechanical-biological waste treatment system (MBA) according to claim 1, **characterised in that** in the processing water circuit (PWK) the ammonium nitrogen content of the processing water (PW) is determined as the nitrogen content (SSG), wherein if necessary liquid (F) is added to the processing water circuit (PWK) in particular upstream of or in the fermentation device (FE).

3. Method for operating a mechanical-biological waste treatment system (MBA) according to claim 1, **characterised in that** the salt content (SZG) of the processing water (PW) is determined downstream of the fermentation device (FE) and upstream of the mechanical separating device (I).

4. Method for operating a mechanical-biological waste treatment system (MBA) according to any one of the preceding claims, **characterised in that** the processing water (PW) in the processing water circuit (PWK) is dammed in at least one mixing and storage device (MSP) in order to reduce concentration peaks in particular of the salt content (SZG) and/or the nitrogen content (SSG) and/or hydraulic load peaks and/or content of organic matter.

5. Method for operating a mechanical-biological waste treatment system (MBA) according to any one of the preceding claims, **characterised in that** the supplied liquid (F) is mixed with the processing water (PW) prior to introduction into the fermentation device (FE) in a mixing and storage device (MSP).

6. Method for operating a mechanical-biological waste treatment system (MBA) according to any one of the preceding claims, **characterised in that** the salt content (SZG) and/or the nitrogen content (SSG) of the processing water (PW) in the mixing and storage device (MSP) is measured.

7. Method for operating a mechanical-biological waste treatment system (MBA) according to any one of the preceding claims, **characterised in that** deposit inhibitors are added to the processing water to avoid deposits in particular in the processing water circuit (PWK) and in particular in the pipes and units guiding the excess processing water (PW).

8. Method for operating a mechanical-biological waste treatment system (MBA) according to any one of the preceding claims, **characterised in that** a solid matter content of the processing water (PW) can be controlled for an optimal operation of the fermentation device (FE) by returning processing water (PW) with a low solids content from the decanter unit (DKE) or by supplying concentrated residual matter.

## Revendications

1. Procédé de fonctionnement d'une installation mécano-biologique de traitement des déchets (MBA) avec un circuit d'eau de traitement (PWK), ladite installation de traitement des déchets (MBA) produisant un excédent d'eau de traitement (PWÜ), comportant
- un dispositif de séparation (I) mécanique, dans lequel les déchets sont séparés en une fraction de déchets majoritairement solide, à savoir la matière solide (FS), et une fraction de déchets majoritairement liquide à teneur organique, à savoir l'eau de traitement (PW),
- un dispositif de traitement (II) à plusieurs niveaux, dans lequel se poursuit le traitement de la matière solide (FS),
- un dispositif de traitement (III) à plusieurs niveaux, qui est exploité parallèlement au dispositif de traitement (II) et dans lequel se poursuit le traitement de l'eau de traitement (PW),
- un gaz (BG) étant produit dans un dispositif de fermentation (FE) du dispositif de traitement (III), tout d'abord à partir de l'eau de traitement (PW) affluant hors du dispositif de séparation (I),
- après son passage à travers le dispositif de fermentation (FE), l'eau de traitement (PW) étant séparée au moins en partie dans un dispositif de décantation (DKE) en matières résiduelles (RST) et en eau de traitement (PW) pauvre en matières solides,
- les matières résiduelles étant évacuées hors de l'installation MBA ou continuant à être traitées en moins en partie dans l'installation MBA,
- un volume excédentaire (PWÜ) de l'eau de traitement (PW) pauvre en matières solides étant évacué hors du circuit d'eau de traitement (PWK) et, en particulier, hors de l'installation MBA,
- un volume résiduel d'eau de traitement (PW) pauvre en matières solides restant dans le circuit d'eau de traitement (PWK),
**caractérisé en ce que**
un liquide (F) est acheminé en fonction de la teneur en sel (SZG) déterminée dans l'eau de traitement (PW) du circuit d'eau de traitement (PWK) et, en vue de diminuer la concentration du chlorure dans le circuit d'eau de traitement (PWK) tout en diminuant parallèlement l'addition de chlorure ferrique (III), on augmente l'addition d'oxyde de fer en tant que moyen de désulfuration, afin de réduire la pénétration du sel, provenant de l'eau de traitement (PW), dans la matière solide (FP),
et un liquide (F) est acheminé en fonction de la teneur en azote (SSG) déterminée dans l'eau de traitement (PW) du circuit d'eau de traitement (PWK), afin de diminuer la teneur en azote dans l'eau de traitement (PW) pour le fonctionnement du dispositif de fermentation (FE).

2. Procédé de fonctionnement d'une installation mécano-biologique de traitement des déchets (MBA) selon la revendication 1, **caractérisé en ce que** dans le circuit d'eau de traitement (PWK) la teneur en azote d'ammonium est déterminée en tant que teneur en azote (SSG), dans l'eau de traitement (PW), un liquide (F) étant acheminé, le cas échéant, vers le circuit d'eau de traitement (PWK), en particulier en amont du dispositif de fermentation (FE) ou à l'intérieur de celui-ci.

3. Procédé de fonctionnement d'une installation mécano-biologique de traitement des déchets (MBA) selon la revendication 1, **caractérisé en ce que** la teneur en sel (SZG) dans l'eau de traitement (PW) est déterminée en aval du dispositif de fermentation (FE) et en amont du dispositif de séparation (I) mécanique.

4. Procédé de fonctionnement d'une installation mécano-biologique de traitement des déchets (MBA) selon l'une quelconque des revendications précédentes, **caractérisé en ce que** l'eau de traitement (PW) dans le circuit d'eau de traitement (PWK) est retenue dans au moins un dispositif de mélange et de stockage (MSP), afin de réduire les pointes de concentration, en particulier pour la teneur en sel (SZG) et/ou pour la teneur en azote (SSG), et/ou des pointes de charge hydrauliques et/ou la teneur en matières organiques.

5. Procédé de fonctionnement d'une installation mécano-biologique de traitement des déchets (MBA) selon l'une quelconque des revendications précédentes, **caractérisé en ce que** le liquide (F) acheminé est mélangé à l'eau de traitement (PW) dans un dispositif de mélange et de stockage (MSP), avant l'admission dans le dispositif de fermentation (FE).

6. Procédé de fonctionnement d'une installation mécano-biologique de traitement des déchets (MBA) selon l'une quelconque des revendications précédentes, **caractérisé en ce que** la teneur en sel (SZG) et/ou la teneur en azote (SSG) dans l'eau de traitement (PW) est mesurée à l'intérieur du dispositif de mélange et de stockage (MSP).

7. Procédé de fonctionnement d'une installation mécano-biologique de traitement des déchets (MBA) selon l'une quelconque des revendications précédentes, **caractérisé en ce que** des inhibiteurs de dépôts sont ajoutés à l'eau de traitement (PW) afin d'empêcher les dépôts en particulier dans le circuit d'eau de traitement (PWK) et en particulier dans les canalisations et les unités dans lesquels circule l'eau de traitement (PW) excédentaire.

8. Procédé de fonctionnement d'une installation mécano-biologique de traitement des déchets (MBA) selon l'une quelconque des revendications précédentes, **caractérisé en ce que**, en vue d'un fonctionnement optimal du dispositif de fermentation (FE), la teneur en matières solides dans l'eau de traitement (PW) peut être réglée par le recyclage de l'eau de traitement (PW) pauvre en matières solides sortant du dispositif de décantation (DKE) ou par l'acheminement de matières résiduelles épaissies.
